Europäisches Patentamt

European Patent Office

Office européen des brevets

(11) Publication number: **0 131 228**
A2

# EUROPEAN PATENT APPLICATION

(21) Application number: **84107643.3**

(22) Date of filing: **02.07.84**

(51) Int. Cl.⁴: **A 61 K 31/44**
**A 61 K 9/06, A 61 K 47/00**

(30) Priority: **08.07.83 JP 124140/83**

(43) Date of publication of application:
**16.01.85 Bulletin 85/3**

(84) Designated Contracting States:
**DE FR GB IT**

(71) Applicant: **YAMANOUCHI PHARMACEUTICAL**
**No. 5-1 Nihonbashi-Honcho, 2-chome Chuo-ku**
**Tokyo(JP)**

(72) Inventor: **Konno, Yutaka**
**21-6, Bessho**
**Omiya-shi Saitama(JP)**

(72) Inventor: **Kawata, Hiroitsu**
**No. 1-7, Oaza Namiki**
**Kawagoe-shi Saitama(JP)**

(72) Inventor: **Aruga, Masayoshi**
**No. 5-16-14, Midorigaoka**
**Ageo-shi Saitama(JP)**

(72) Inventor: **Sonobe, Takashi**
**No. 4-1-12, Fujimi Fukiage-cho**
**Kitaadachi-gun Saitama(JP)**

(72) Inventor: **Takenaka, Toichi**
**No. 3-27-H-1108, Nakadai Itabashi-ku**
**Tokyo(JP)**

(74) Representative: **Wuesthoff, Franz, Dr.-Ing. et al,**
**Patentanwälte Wuesthoff -v. Pechmann-Behrens-Goetz**
**Schweigerstrasse 2**
**D-8000 München 90(DE)**

(54) **Nicardipine hydrochloride or nifedipine ointments.**

(57) An ointment containing nicardipine hydrochloride or nifedipine and a mixed solvent of benzyl alcohol and propylene glycol or a mixed solvent of N-methyl-2-pyrrolidone and propylene glycol together with an emulsifier and a base. Furthermore, an ointment having the above-described composition further containing a penetration enhancer.

EP 0 131 228 A2

Croydon Printing Company Ltd.

NICARDIPINE HYDROCHLORIDE OR NIFEDIPINE OINTMENTS

FIELD OF THE INVENTION

This invention relates to an ointment containing nicardipine hydrochloride or nifedipine. More particularly, the invention relates to an ointment containing nicardipine hydrochloride or nifedipine and a mixed solvent of benzyl alcohol and propylene glycol or a mixed solvent of N-methyl-2-pyrrolidone and propylene glycol together with an emulsifier and a base. Furthermore, the invention relates to an ointment having the abovedescribed composition further containing a penetration enhancer.

BACKGROUND OF THE INVENTION

Recently, a systemic treatment by the transdermal administration of a medicament such as nitroglycerin, scopolamine, isosorbitol dinitrate, etc., has been tried. The transdermal administration has several advantages like simple way for administration of drug, a long duration, less occurence of side effect, avoiding passage through the liver before distribution, etc., and is one of administration methods the further application and development of which have been expected.

Nicardipine hydrochloride is a very useful novel compound having a cerebral vascular dilator activity, a coronary dilator activity, and an anti-hypertension activity but the compound has been practically used as dosage forms for oral administration only but dosage forms administrated percutaneously using the compound has not yet been developed. Furthermore, nifedipine is also a compound having a coronary dilator activity and an antihypertension activity and the dosage forms for oral administration only using the compound has been on the market but the dosage forms administrated percultaneously have not yet been on the market.

Considering the above-described medicinal actions, the transdermal administraion of nicardipine hydrochloride or nifedipine can become a very effective administration method. However, dosage forms administrated percutaneously have a difficultly in the absorption of medicament and hence this dosage forms have now been utilized for limited medicaments only. Consequently, the development of the rational design of topical formulations for nicardipine hydrochloride or nifedipine has been demanded.

## SUMMARY OF THE INVENTION

As the result of various investigations on the development of dosage forms administrated percutaneously, in particular an ointment of nicardipine hydrochloride or nifedipine from the above-described view point, the inventors have discovered that an ointment prepared by incorporating nicardipine hydrochloride or nifedipine, a mixed solvent of benzyl alcohol and propylene glycol or a mixed solvent of N-methyl-2-pyrrolidone and propylene glycol, an emulsifier and a base is excellent in percutaneous absorption of nicardipine hydrochloride or nifedipine. Furthermore, it has been discovered that by further adding an penetration enhancer to the above-described compounded components, the ointment having a more imporoved percutaneous absorption of the medicament. Based on these discoveries, the invention has been attained.

The components described above can be practically used for other topical formulations such as a plaster or patch form by combining with a proper adhesive, etc.

DESCRIPTION OF THE PREFERRED EMBODIMENT

Now, benzyl alcohol, N-methyl-2-pyrrolidone, and propylene glycol which are used in this invention are solvents for the active substance and among them, benzyl alcohol and N-methyl-2-pyrrolidone have particularly high dissolving property for the active substance and also propylene glycol has a high absorption accelerating action for the active substance. By compounding these solvents with nicardipine hydrochloride or nifedipine as a combination of benzyl alcohol and propylene glycol or a combination of N-methyl-2-pyrrolidone and propylene glycol, the percutaneous absorption thereof is unexpectedly increased. Also, there is no particular restriction about the amount of the solvents if the active substance is homogeneously dispersed therein and the amount of the solvents can maintain the mixed components as an ointment form, but the proper amount of the solvents is usually 1 to 20% by weight.

As the emulsifiers which can be used in this invention, there are nonionic surface active agents such as glycerol higher fatty acid esters (e. g., Nikkol MGS-B: trade name, made by Nikko Chemical Co., Ltd.), sorbitan fatty acid esters (e. g., Span 60: trade name, made by Kao Atlas Co., Ltd.), polyoxyethylene-hardened castor oil derivatives (e. g., Nikkol HCO-60: trade name, made by Nikko Chemical Co., Ltd.), etc. The proper amount of the emulsifier is 0.5 to 10% by weight.

There is no particular restriction about the base which is used in this invention but oily bases such as white vaselin, plastibases, silicone oils, etc., are particularly preferred.

As the penetration enhancers which are used in this invention, there are fatty acid esters such as diisopropyl adipate, diethyl adipate, diethyl sebacate, dibutyl sebacate, diethyl glycol adipate, ethyl laurate, isopropyl myristate, isopropyl palmitate, etc.; urea; various kinds of surface active agents, etc. These absorption accelerators may be used solely or as a combination of two or more. The amount of the absorption accelerator is usually 0.5 to 20% by weight, preferably 5 to 15% by weight.

In addition, propylene glycol which is used as a solvent in this invention also has an absorption promoting action and hence in the case of using propylene glycol as one of the solvents, there is an advantage of accelerating the absorption of the active substance when the above-described penetration enhancer is not used.

The ointment of this invention can be prepared according to an ordinary method of preparing ointments. For example, the ointment is prepared by dissolving nicardipine hydrochloride or nifedipine in a mixed solvent of benzyl alcohol or propylene glycol or a mixed solvent of N-methyl-2-pyrrolidone and propylene glycol under heating to about 40°C, gradually adding the soltution to a mixture of an ointment base and an emulsifier , which was melted at about 70°C and then cooled to room temperature, and sufficiently kneading the mixture until a homogeneous mixture was obtained. Also, the case of adding an absorption accelerator, nicardipine hydrochloride or nifedipine and the absorption accelerator are dissolved in the above-described mixed solvent under heating and thereafter, the same procedure as above is followed.

In addition, the ointment of this invention may further contain other ajuvants such as a middle fatty acid and the middle chain fatty acid triglyceride thereof (e.g., ODO; trade name, made by Nisshin Seiyu K. K.), an unsaturated higher fatty acid (e. g., oleic acid, linolic acid, etc.,), white wax, solid paraffin, etc.

Then, percutaneous absorption experiments about the ointments of this invention and the results thereof are show below.

(1). Percutaneous absorption experiment about beagle dog:

i) Experimental procedure:

The abdomen of a beagle dog having a weight of about 13 kg was shaved by means of an electric clipper and a depilatory cream, the ointment (100 mg as nicardipine) was spread on Parafilm (made by American Can Co.) of 10 x 10 $cm^2$ in area, the film was stuck to the shaved skin of the dog, and the film was fixed by means of a surgical tape and further by a gum tape. After 1, 3, 5, 7, and 9 hours since the transdermal administration, blood was collected and the plasma level of nicardipine hydrochloride was measured by Higuchi et als' method (J. Chromatogr., 110, 301(1975)).

ii) Result:

| Sample | Plasma concentration of nicardipine hydrochloride (ng/ml) | | | | |
|---|---|---|---|---|---|
| | 1H | 3H | 5H | 7H | 9H |
| Example 1 | 0 | 3.0 | 6.0 | 5.5 | 4.5 |
| Example 2 | 0 | 6.0 | 11.0 | 10.5 | 11.5 |
| Contrast* | 0 | 0 | 0 | 0 | 0 |

(*): Ointment prepared by adding 2.5 g of nicardipine hydrochloride and 97.5 g of white wax and sufficiently kneading the mixture.

(2) Percutaneous absorption experiment about guinea pig:

i) Experimental procedure:

The back portion of a guinea pig having a weight of about 400 g was shaved by means of an electric clipper and a depilatory cream. Each of the ointments obtained in Examples 5, 9 and 10 described hereinafter (22-25 mg equivalent to nicardipine hydrochloride) was placed on a surgical tape and the tape was applied to the shaved skin of the guinea pig. Thereafter, the tape was softly pushed by a finger so that the area of the ointment became about 10 $cm^2$. After 5 hours,

the blood was collected at the same time and the plasma concentration of nicardipine hydrochloride was measured according to the Higuchi et als' method (J. Chromatogr., 110, 301(1975)).

ii) Result:

| Sample | Plasma concentration (ng/ml) |
| --- | --- |
| Example 5 | 37.6 |
| Example 9 | 40.0 |
| Example 10 | 16.8 |
| Control * | 0 |

(*): An ointment prepared by adding 2.5 g of nicardipine hydrochloride to 97.5 g of white wax and sufficiently kneading the mixture.

iii)  Other result:

By following  the procedure as in above-mentioned experiment, testing other some samples, following result was obtainted.

| Sample | Plasma concentration (ng/ml) |
|---|---|
| Example 17 | 45.8 |
| Example 20 | 30.0 |
| Control (a) | 5.7 |
| Control (b) | 16.8 |

Then, the invention will further be explained by the following examples.

### Example 1

A mixture of 82.5 g of plastibase and 5 g of glyceryl monostearate (Nikkol MGS-B) was melted at about 80°C and was transferred in a kneader. A solution of 2.5 g of nicardipine hydrochloride dissolved in a mixture of 5 g of benzyl alcohol and 5 g of propylene glycol was gradually added to the melt with stirring and the mixture was sufficiently kneaded to provide an ointment.

### Example 2

By following the same procedure as in Example 1 using white wax in place of plastibase, an ointment was obtained.

### Example 3

A mixture of 77.5 g of white vaseline and 5 g of glyceryl monostearate (Nikkol MGS-B) was melted at about 70°C and was transferred to a kneader. A solution of 2.5 g of nicardipine hydrochloride dissolved in a mixture of 5 g of benzyl alcohol, 5 g of propylene glycol, and 5 g of diisopropyl adipate was gradually added to the melt and the mixture was sufficiently kneaded to provide an ointment.

### Example 4

By following the same procedure as in Example 3 using sorbitan monostearate (Span 60) in place of glyceryl mononstearate, an ointment was obtained.

### Example 5

By following the same procedure as in Example 3 using a polyoxyethylene castor oil derivative (Nikkol HCO-60) in place of the glyceryl monostearate in Example 3, an ointment was obtained.

### Example 6

By following the same procedure as in Example 3, N-methyl-2-pyrrolidone was used in place of benzyl alcohol , an ointment was prepared.

### Example 7

A mixture of 75 g of white vaseline, 5 g of sorbitan monostearate (Span 60) and 2.5 g of isopropyl myristate was melted at about 70°C and transferred into a kneader. A solution of 2.5 g of nicardipine hydrochloride dissolved in a mixture of 5g of benzyl alcohol, 5 g of propylene glycol, and 5 g of diisopropyladipate was added gradully to the melt under stirring followed by sufficiently kneading to provide a semi-solid ointment.

## Example 8

A mixture of 72.5 g of white vaseline, 5 g of sorbitan monostearate (Span 60) and 5 g of ODO (triglyceride of a middle fatty acid, trade name, made by Nosshin Seiyu K. K.) was melted at about 70°C and was transferred into a kneader. A solution of 2.5 g of nicardipine hydrochloride dissolved in a mixture of 5 g of benzyl alcohol, 5 g of propylene glycol and 5 g of diisopropyl adipate was gradually added to the melt under stirring and the mixture was sufficiently kneaded to provide an ointment.

## Example 9

By following the same procedure as Example 7 using a polyoxyethylene castor oil derivative (Nikkol HCO-60) in place of sorbitan monostearate, an ointment was obtained.

## Example 10

By following the same procedure as Example 7 using a polyoxyethylene castor oil derivative (Nikkol HCO-60) in place of sorbitan monostearate and linolic acid in place of ODO, an ointment was obtained.

## Example 11

A mixture of 5 g of white vaseline, 5 g of glyceryl monostearate (Nikkol MGS-B) and 5 g of isopropyl myristate was melted and after adding thereto 67.5 g of a silicone oil (MDX-4-4210, trade name, made by Dow Corning Co.), the mixture was kneaded well in a kneader. A solution of 2.5 g of nicardipine hydrochloride dissolved in a mixture of 5 g of benzyl alcohol, 5 g of propylene glycol and 5 g of diisopropyl adipate under heating was gradually added to the mixture and the resultant mixture was kneaded well to provide an ointment.

## Example 13

A mixture of 77.5 g of white vaseline and 5 g of a polyoxyethylene-hardened castor oil derivative (Nikkol HCO-60) was melted at about 70°C and was transferred into a kneader. A solution of 2.5 g of nifedipine dissolved in a mixture of 5 g of N-methyl-2-pyrrolidone, 5 g of propylene glycol and 5 g of diisopropyl adipate was gradually added to the melt with stirring and the mixture was sufficiently kneaded to provide an ointment.

## Example 14

By following the same procedure as Example 13 using plastibase in place of white vaseline, an ointment was obtained.

## Example 15

By following the same procedure as Example 13 using a silicone oil in place of white vaseline, an ointment was obtained.

## Example 16

A mixture of 79 g of white vaseline and 5 g of polyoxyethylene-hardened castor oil derivative (Nikkol HCO-60) was melted at about 70°C and was transferred into a kneader. A solution of 1 g of nifedipine dissolved in a mixture of 5 g of benzyl alcohol, 5 g of propylene glycol and 5 g of diisopropyl adipate was gradually added to the melt with stirring and the mixture was sufficiently kneaded to provide an ointment.

## Example 17

A mixture of 150 g of white vaseline 9.1 of glycerine monostearate (NIkkol MGS-B) was melted at about 70°C and was transferred into a kneader. A solution of 4.5 g of nicardipine hydrochloride dissolved in a mixture of 9.1 g of benzyl alcohol and 9.1 g of propylene glycol was gradually added to the melt with stirring. Finally, 18.2 g of an aqueous 50% (w/w) of urea was gradually added to the mixture with stirring and the resultant mixture was sufficiently kneaded to provide an ointment.

## Example 18

By following the same procedure as Example 17 using N-methyl-2-pyrrolidone in place of benzyl alcohol, an ointment was obtained.

## Example 19

A mixture of 150 g of white vaseline and 9.1 g of glyceryl monostearate (Nikkol MGS-B) was melted and was transferred into a kneader. A solution of 4.5 g of nifedipine dissolved in a mixture of 9.1 of N-methyl-2-pyrrolidone and 9.1 g of propylene glycol was gradually added to the melt with stirring. Finally, 18.2 g of an aqueous solution of 50% (w/w) of urea was added to the mixture with stirring and the resultant mixture was sufficiently kneaded to provide an ointment.

Example 2c

A mixture of 159.1 g of white vaseline and 9.1 g of glyceryl monostearate (Nikkol MGS-B) was melted at about 60°C and was transferred into a kneader. A solution of 4.5 g of nicardipine hydrochloride dissolved in a mixture of 9.1 g of benzyl alcohol and 9.1 g of propylene glycol was gradually added to the melt with stirring. Finally, 9.1 g of pulverized urea was added to the mixture and the resultant mixture was sufficiently kneaded to provide an ointment.

Control (a)

A mixture of 186.4 g of white vaseline and 9.1 g of glyceryl monostearate (Nikkol MGS-G) was melted at about 60°C and was transferred into a kneader. 4.5 g of nicardipine hydrochloride was directly added to the melt and the mixture was sufficiently kneaded to provide an ointment.

Control (b)

A mixture of 177.3 g of white vaseline and glyceryl monotearate (Nikkol MGS-B) was melted at about 60°C and was transferred into a kneader. A solution of 4.5 g of nicardipine hydrochloride dissolved in 9.1 g of benzyl alcohol was gradually added to the melt with stirring and the mixture was sufficiently kneaded to provide an ointment.

CLAIMS:

1. An ointment comprising nicardipine hydrochloride or nifedipine; a mixed solvent of benzyl alcohol and propylene glycol or a mixed solvent of N-methyl-2-pyrrolidone and propylene glycol; an emulsifier and a base.

2. An ointment comprising nicardipine hydrochloride or nifedipine; a mixed solvent of benzyl alcohol and propylene glycol or a mixed solvent of N-methyl-2-pyrrolidone and propylene glycol; an emulsifier; a penetration enhancer and a base.

3. The ointment as claimed in claim 1 or 2, wherein the base is white vaseline, plastibase or a silicone oil.

4. The ointment as claimed in claim 1 or 2, wherein the base contains white vaseline, plastibase or a silicone oil.

6238